# EUROPEAN PATENT SPECIFICATION

(11) **EP 3 986 421 B1**
(45) Date of publication and mention of the grant of the patent: **01.04.2026**
(21) Application number: 20735028.1
(22) Date of filing: 22.06.2020
(51) Int. Cl.: A61K 31/728, A61L 27/20, A61L 27/50, A61L 27/52, A61P 19/08

(54) **PHARMACEUTICAL COMPOSITIONS COMPRISING AMIDE DERIVATIVES OF HYALURONIC ACID, FOR USE IN THE TREATMENT OF BONE TRAUMAS, IN PARTICULAR OF PATIENTS WITH PROBLEMS OF OSTEOPENIA OR OSTEOPOROSIS**
PHARMAZEUTISCHE ZUSAMMENSETZUNGEN MIT AMIDDERIVATEN VON HYALURONSÄURE ZUR VERWENDUNG IN DER BEHANDLUNG VON KNOCHENTRAUMEN, INSBESONDERE VON PATIENTEN MIT PROBLEMEN DER OSTEOPENIE ODER OSTEOPOROSE
COMPOSITIONS PHARMACEUTIQUES COMPRENANT DES DÉRIVÉS AMIDIQUES D'ACIDE HYALURONIQUE, DESTINÉES À ÊTRE UTILISÉES DANS LE TRAITEMENT DE TRAUMATISMES OSSEUX, EN PARTICULIER DE PATIENTS PRÉSENTANT DES PROBLÈMES D'OSTÉOPÉNIE OU D'OSTÉOPOROSE

(30) Priority: 24.06.2019 IT 201900009933
(43) Date of publication of application: 27.04.2022
(73) Proprietor: Fidia Farmaceutici S.p.A., 35031 Abano Terme (PD) (IT)
(72) Inventor: LISIGNOLI, Gina, 40136 BOLOGNA (IT); PIZZOCARO, Carlo, 35031 Abano Terme (PD) (IT)
(74) Representative: Bird & Bird Società tra Avvocati S.r.l.
(86) International application number: PCT/IB2020/055848
(87) International publication number: WO 2020/261082

(56) References cited:
- WO-A1-2006/092233
- WO-A1-2013/179258

## Description

The present invention relates to pharmaceutical compositions comprising amide derivatives of hyaluronic acid for use in the treatment of bone mineralization in bone traumas, in particular in patients with problems of osteopenia or osteoporosis.

### STATE OF THE ART

The regeneration of bone tissue represents an extremely interesting field of research; the possibility of growing bone tissue *ex-novo* is in fact crucial not only for some very particular applications aimed at bone reconstruction, such as for example dental implantology, the filling of cavities caused by traumas, surgery, diseases, but also for facilitating the ossification and subsequent remodelling of fractures, or lesions in a wider sense, that affect patients with bone fragility, assessed according to appropriate parameters.

Briefly, ossification is the end point of a series of events thanks to which the mesenchymal tissue is transformed into bone tissue through mineralization. Bone mineralization consists in filling the extracellular matrix with mineral crystals, substantially microcrystals/nanocrystals of calcium phosphate, in an organized way and according to a well-defined sequence of events. The cells responsible for the mineralization activity are osteoblasts, which derive from the differentiation of the cells contained in the mesenchymal tissue, as described in greater detail hereunder.

Ossification substantially occurs in two ways:
- intramembranous ossification, or direct, responsible for the formation of short bones and flat bones, which, for this reason, are called membranous; among these are the jaw, the clavicle and the bones of the skullcap, i.e. bones that do not have a supporting function; the transition from mesenchymal to bone tissue is direct;
- intra-chondral ossification, or indirect, which forms the so-called cartilaginous bones: these are generally long bones, such as those of the lower limb and the upper limb or the vertebrae, or bones with a supporting function. In this case, the transition from mesenchymal to bone tissue is mediated by a cartilaginous phase.

In both cases, ossification is a slow mineralization process, i.e. the continuous deposition of mineral crystals, and parallel remodelling, i.e. reabsorption of the material deposited, with a deposition rate which in a fetus and in a growing organism, is greater than its reabsorption rate; in an adult, under physiological conditions, the two processes have the same rate. In adults, the bone formed is called lamellar, specifically because it is organized in very thin overlapping laminae.

As already specified, the mesenchymal cells play a decisive role in these complex phases: it is the mesenchymal cells, in fact, that under the biochemical thrust of growth factors, hormones and cytokines brought by the blood flow into the extracellular matrix in which they are immersed, differentiate into osteoblasts: the latter aggregate in dense structures, initiate the mineralization phase and, through further very complex mechanisms, allow the formation of bones, finally transforming into osteocytes, quiescent within the bone gaps.

Together with the mineralization activity, osteoblasts have the task of activating the release of osteoclasts, in turn responsible for bone remodelling, following a hormonal mechanism mediated by interaction with the parathyroid hormone. And it is specifically on the balance between mineralization and remodelling that the formation and maintenance of the bone structure are based.

Given the complexity of these processes, it is clear that the bone structure can be compromised and made particularly weak by a wide range of factors, among which the following should be remembered:
- diseases, genetic or metabolic; for example, diabetes, Gaucher disease, osteomalacia, rickets, primary postmenopausal or elderly osteoporosis, osteoporosis secondary to chronic drug intake, for example steroids, chemotherapy, immunosuppressants, GnRH analogues;
- hormonal imbalances and related pathologies, for example, hypogonadism, hyperparathyroidism, Cushing's disease;
- pathological deficiencies of calcium, phosphorus, vitamin D3 (avitaminosis) due to caloric-protein malnutrition, low body weight, malabsorption syndrome.

Furthermore, it should not be forgotten that the bone metabolism is strongly influenced by an incorrect lifestyle, such as physical inactivity, limited exposure to sunlight, excess smoking and alcohol (Lane NE, Am. J. Obstet. Gynecol., 2006, 194, S3 -11; Marcucci et al., Clin. Cases Min. Bone Metab., 2015, 12, 151-6).

The factors indicated above, associated with an increase in the average age of the population, entail the occurrence of an increasing number of situations in which the presence of bone fragility leads to fractures or injuries that have difficulty in mineralizing and remodelling, forcing the patient to remain immobile or otherwise profoundly altering his/her quality of life.

The fundamental criterion for defining a situation of bone fragility, regardless of the causes that induced it, is the evaluation of bone mineral content (BMC) and bone density (BMD); the quantity and density of mineral salts contained in the skeletal region examined are therefore measured with a special instrument that uses X-rays in very low quantities (Bone Mineralometry Computerized with Dual X-ray Absorptiometry technique). The analysis can be carried out at the level of the forearm, the lumbar spine, the femur or the whole skeleton. In most cases it is effected at the level of the spine or the femur.

In an adult person, the diagnosis is effected by examining the "T-score", i.e. by assessing to what extent the BMD value in question differs from that of the reference sample (healthy subjects of the same sex and aged 25-30 years, i.e. examined when the peak of bone mass is reached).

T-score values ranging from +1 to -1 indicate normal bone mineralization.

According to the criteria of the World Health Organization (originally referring to menopausal women, but today used for adults of both sexes), osteopenia is the case when the T-score value is less than -1, and osteoporosis when the T-score is less than -2.5 (Assessment of fracture risk and its application to screening for postmenopausal osteoporosis. Report of a WHO Study Group. Geneva, World Health Organization, 1994 (WHO Technical Report Series, No. 843)).

The bone mass value measured is strongly indicative of the general health of the skeleton. Patients with a T-score lower than -1 are obviously exposed to a greater risk of fractures and have greater difficulty in mineralizing the bone callus, necessary for healing the fracture; these patients, in fact, are generally subjected to therapies (Calcium, Vitamin D) which attempt to improve bone mineralization.

The repair of a fracture generally takes place according to the following stages:
- reduction in the hematoma: the blood flowing to the fracture point forms a clot that isolates the damaged area, helps stabilize broken bone margins and simultaneously provides growth factors, cytokines and whatever is necessary for the
- activation of the mesenchymal cells: in a mature bone they are essentially concentrated in the periosteum, which lines the outside of the bone, and in the endostium, which lines the medullary cavity, i.e. the internal surface of the bone. Breakage of the bone causes their release;
- the mesenchymal cells, activated by the set of factors that arrive in the extracellular matrix in which they are immersed, differentiate into osteoblasts, which begin to produce further extracellular matrix and the so-called soft periosteal bone callus, which acts as a bridge between the fractured ends and stabilizes free bone fragments, if present. After the formation of this basic structure, the osteoblasts begin the mineralization phase, and therefore the deposition of microcrystals/calcium phosphate nanocrystals; the osteoclasts, suitably stimulated, subsequently activate the remodelling. In this way, following the alternation of deposition and parallel bone resorption, the bone callus is gradually replaced by actual lamellar bone. The osteoblasts, now transformed into osteocytes, settle in the bone gaps, in a state of quiescence.

When the lesion is small and composed, i.e. if it is a lesion in which the fractured ends are stabilized and perfectly match, there is primary healing, in which the mesenchymals differentiated into osteoblasts mineralize and begin the deposition of bone without the formation of bone callus.

Regardless of the type of fracture and the type of bone involved, the healing phases have a very variable duration which depends on the type of fracture, the type of bone, the general condition of the patient, his/her age, etc.

In principle, in a healthy adult, the phases of hematoma reduction, mesenchymal stimulation and production of soft bone callus take about 2 weeks, ossification of the callus takes place in 4-6 weeks following the fracture and the remodelling phase, in which the bone acquires its definitive form of mature (lamellar) bone, generally begins 6-7 weeks after the fracture and can even last a few years.

In the case of fragile bones, weakened by the causes or factors already discussed, in patients with T-score <-1, these times can naturally be extremely prolonged, with easily predictable consequences.

It is therefore important to identify a treatment that activates and improves bone mineralization for restorative purposes both in subjects for whom, having for the various causes already described a T-score <-1, this process is compromised (for example, osteopenic subjects, with a T- score between -1 and -2.5 or, even worse, osteoporotics, with a T-score <-2.5), and also in healthy subjects for whom fast bone regeneration is desirable (for example, injured athletes). A stimulus to repair may be necessary not only as a result of a fracture, but also in all those traumatic situations that could benefit from fast and effective ossification; these range from the most impressive traumas, for example with a significant loss of bone material, to maxillofacial reconstructive surgery, and to minor traumas (for example, dental reconstruction).

The region on which to act for this purpose is definitely represented by periosteal and endostal mesenchymal cells, the key point of bone mineralization.

There are numerous methods that are most widely studied and followed known in the state of the art for the activation of the mesenchymal cells; for example, scaffolds of various materials are being studied, which release specific osteogenic factors (Huang et al., J. Bone Miner. Res., 2005, 848-857; Karageorgiou K., J. Biomed. Mater. Res., 2004 , 528-537). Attempts are also known aimed at systemically administering hormone-associated mesenchymal cells (Yakubovich 2017), or topically administering growth factors (Schmidmaier 2002).

A further approach is that which involves using one of the fundamental components of the extracellular matrix locally, i.e., hyaluronic acid, in the form of a three-dimensional construct colonizable by cells (Ayanoglu et al., Acta Orthop. Traumatol. Turc., 2015, 49, 319-325), or as such (Zou et al., J. Orthoped. Res., 2008, 713-720). Finally, the possibility of using biological materials consisting of mesenchymal cells of various origins conveyed by a solution of hyaluronic acid (WO2011000820) is known; the dynamic viscosity of these preparations is extremely low and absolutely unsuitable for the applications provided for within the scope of the invention described herein, as the preparation is not sufficiently viscous to remain on site and slips away from the application site.

WO 2013/179258 Al discloses a composition comprising 8 mg/mL of hyaluronic acid (with MW 500 and 730 kDa) amide with hexadecylamine having a molar amidation percentage ranging between 8 and 9% and a protein, preferably selected from insulin, hGH, sCT, IL-IRa, G-CSF, PDGF, preferably PDGF-BB, TGF-β, EGF, VEGF-B, EPO, NGFp, transferrin, TIMP-2, TIMP-3 and TIMP-4, for use in the repair of bone fractures.

The present invention overcomes the drawbacks and problems of the state of the art, making particular pharmaceutical compositions available for use in the treatment of bone traumas, preferably bone fractures, in particular in patients suffering from pathologies characterized by a T-score index lower than -1 and, in particular, lower than -2.5, i.e. for example in patients with problems of osteopenia or osteoporosis.

These compositions can be administered locally, locoregionally, intraosseously, perioxially, but not intraarticularly, they may possibly contain pharmacologically and biologically active substances, and can be formulated with pharmaceutically acceptable excipients known to persons skilled in the art. They have proved to be active in promoting bone mineralization and therefore promoting the ossification process.

### DESCRIPTION

The present invention therefore relates to a pharmaceutical composition as defined in claim 1.

The pharmaceutical compositions for use according to the present invention are used in the treatment of bone traumas, preferably bone fractures, in patients suffering from pathologies characterized by a T-score index lower than -1, preferably lower than -2.5.

The hyaluronic acid (HA) derivative of the pharmaceutical composition for the use according to the present invention is the hexadecyl amide prepared from a HA having a weight average MW ranging from 500 kDa to 730 kDa, and having an amidation degree ranging from 1% to 3% m/m. When the amidation degree ranges from 1% to 3% m/m, the dynamic viscosity ranges from 55 to 75 Pa·s.

Thanks to the high viscosity and also the natural mucoadhesive properties of hyaluronic acid, the pharmaceutical compositions for use according to the present invention remain stably in the application site and are particularly suitable for administration routes such as local, locoregional, intra- or peri-osseous; conversely, intraarticular administration is excluded.

The compositions are formulated with the addition of pharmaceutically acceptable excipients, identified by skilled persons in the field in relation to the administration site and route.

The main advantage of the pharmaceutical compositions for use according to the present invention is that they exceed the state of the art, stimulating the differentiation in an osteogenic sense of the mesenchymal cells and consequent mineralization surprisingly more consistently than in the case of the use of HA alone. Said composition, moreover, equally surprisingly, activates the expression of some genes that contribute to the deposition and organization of the extracellular bone matrix, thus contributing to the neoformation of bone.

HA is a hetero-polysaccharide composed of alternating residues of D-glucuronic acid and N-acetyl-D-glucosamine, with a linear chain, having a molecular weight that can range from 50,000 to 13 x 10⁶ Da, depending on the source from which it is obtained and the preparation methods used. Hyaluronic acid is practically ubiquitous within the human body, where it plays an important role as a mechanical support for the cells of many tissues such as the skin, tendons, muscles, cartilage, bones, especially long bones, in which it constitutes 3% of the glycosaminoglycans present. Hyaluronic acid is also capable of interacting with cells through its CD44 membrane receptor and activating and/or regulating the migration, proliferation, aggregation and cell differentiation processes, also at a skeletal level. Thanks to the numerous functional groups with which it is provided, the possibility of chemically derivatizing hyaluronic acid in order to obtain structures that preserve the chemical-physical properties of the starting molecule, has been known for some time, acquiring new specific characteristics.

Among the numerous derivatives that can be obtained, particularly important for the purposes of the present invention, are amide derivatives, i.e. the amides between the carboxyl of HA and the amine group of amines of the aliphatic, arylaliphatic, cycloaliphatic, aromatic, cyclic and heterocyclic series, with an amidation degree ranging from 0.1 to 50% and prepared from HA having a weight average MW ranging from 500 to 730 kDa, as described in EP1095064.

Average Molecular Weight (MW) refers to the weight average MW calculated with the intrinsic viscosity method (Terbojevich et al., Carbohydr. Res., 1986, 363-377).

In particular, the pharmaceutical composition for the use according to the present invention comprises the hexadecyl amide prepared from a HA having a weight average MW ranging from 500 kDa to 730 kDa, with an amidation (derivatization) degree ranging from 1% to 3% m/m, detected by HPLC after hydrolysis of the amide and conjugation of the hexadecylamine released with a fluorophoric substance.

The preparation of hexadecyl amide with an average amidation (derivatization) degree of 1-3% m/m is described in EP1853279; this derivative, hereinafter referred to as HYADD^{®}-4, is commercially available, at a concentration of 8 mg/ml in a saline solution, with the trade-name Hymovis^{®} and has long been used for intraarticular visco supplemental therapy for osteoarthritis.

As already specified, one of the fundamental characteristics of the pharmaceutical compositions for use according to the present invention is the high viscosity: the parameter specifically considered is the dynamic viscosity, i.e. the shear stress that a fluid produces between two sliding planes, one of which is stationary. For the pharmaceutical compositions in the form of hydrogels for use according to the present invention, the dynamic viscosity is measured at a temperature of 20 ± 0.5 °C with an Anton Paar MCR 92 rheometer (or equivalent instrument) equipped with a plate-cone measuring device with a 1° angle and a speed ramp with constant acceleration from 0 to 5 sec⁻¹ in 8 minutes, with interpolation of the viscosity values at 1.0 sec⁻¹ (shear rate).

The pharmaceutical compositions in the form of hydrogels for use according to the present invention, i.e. hydrogels wherein the amide used is hexadecyl amide with an amidation degree ranging from 1% to 3% m/m, the dynamic viscosity ranges from 55 to 75 Pa·s. at the indicated conditions. Even more particularly, the hexadecyl amide with an amidation degree ranging from 1% to 3% m/m is used at a concentration ranging from 6 to 9 mg/ml, even more preferably at a concentration equal to 8 mg/ml.

These are consequently much higher dynamic viscosity values than those known; for purely informative purposes, it is specified that the biological materials described in WO2011000820, already mentioned, have a definitely lower dynamic viscosity, being viscous solutions and not hydrogels.

The dynamic viscosity values of the pharmaceutical compositions in the form of hydrogels for use according to the present invention are indicative of compact hydrogels and therefore capable of remaining in the application site. The compositions in the form of hydrogels also preserve the mucoadhesive characteristics typical of the starting polymer (HA) and thanks to this they remain easily in situ after application.

The present invention therefore claims pharmaceutical compositions as defined in claim 1 for use in the treatment of bone mineralization in bone fractures, in particular in patients suffering from pathologies characterized by a T-score index lower than -1 and in particular lower than -2.5, with local, locoregional, intra- or peri-osseous administration, excluding intra-articular administration, wherein the amide derivative is hexadecyl amide with an amidation degree ranging from 1% to 3% m/m and a dynamic viscosity ranging from 55 to 75 Pa·s.

Treatment with HYADD^{®}-4 therefore represents a surprisingly effective therapeutic strategy in stimulating and maintaining the complex biological system necessary for bone mineralization, over time and thus in improving and accelerating the ossification process.

The data presented in the following examples, in fact, demonstrate that the pharmaceutical compositions for use, subject of the present invention are particularly suitable for use in the treatment of bone mineralization in bone traumas, preferably bone fractures, in particular in patients suffering from pathologies such as:
- diseases, genetic or metabolic; for example, diabetes, Gaucher disease, osteomalacia, rickets, primary postmenopausal or elderly osteoporosis, osteoporosis secondary to the chronic intake of drugs, for example steroids, chemotherapy, immunosuppressants, GnRH analogues;
- hormonal imbalances and related pathologies, for example, hypogonadism, hyperparathyroidism, Cushing's disease;
- pathological deficiencies of calcium, phosphorus, vitamin D3 (avitaminosis) due to caloric-protein malnutrition, low body weight, malabsorption syndrome
characterized by a T-score index lower than -1, and in particular lower than -2.5, and with local, locoregional, intra- or peri-osseous administration, excluding intra-articular administration.

The compositions are formulated with the possible addition of pharmaceutically acceptable excipients, identified by skilled persons in the field in relation to the location and administration route.

The following examples are now provided for a better illustration of the invention, which should be considered as illustrative and non-limiting of the same.

### EXAMPLE 1

### Evaluation of MSC mineralization

The *in vitro* effect was studied on the mineralization of mesenchymal stem cells (MSCs) obtained from human bone marrow treated with HYADD^{®}-4 and with hyaluronic acid in the form of unmodified sodium salt (HA) (weight average MW 500-730 kDa), at different concentrations.

### Materials and methods

The MSCs were isolated from bone marrow aspirate from adult and healthy donors, as described in Manfredini et al. (J. Cell. Physiol., 2011, 226, 2675-82). The MSCs were then seeded in monolayer (24,000 cells/cm²) in 24-well plates and cultured in α-MEM containing FBS (Fetal Bovine Serum) at 15% and penicillin-streptomycin 100U/mL-100µg/mL (Sigma-Aldrich) at 37°C, in the presence of 5% CO₂. After 24 hours, the medium was removed and the cells were divided into three groups, each with the addition of a culture medium containing osteoinductive factors (α-MEM, FBS 10%, 100 mM ascorbic acid; 0.1 mM dexamethasone; 10mM β-glycerophosphate - Sigma Aldrich). The cells were treated for up to 28 total days as described hereunder:
Group 1 (negative control): no treatment. The cells were simply kept in culture in the osteoinductive medium;
Group 2: the cells were treated with HYADD^{®}-4 0.5mg/mL;
Group 3: the cells were treated with HA (PM 500-730kDa) 1 mg/mL.

The cell cultures were analyzed at different times (14, 21 and 28 days).

The mineralization was assessed using the Alizarin Red S assay: in short, the cells washed with PBS were fixed for 15 minutes at room temperature in 10% formaldehyde in PBS, washed twice with PBS, stained with an Alizarin Red S solution 40 mM (Sigma-Aldrich) at pH 4.2 at room temperature for 20 minutes, and finally washed with deionized water in an orbital shaker at 40 rpm to reduce non-specific binding. The Alizarin Red S dye binds to the calcium ions present in the mineralized deposits and creates a bright red colour. The absorbance of each well was read at a wavelength of 510 nm using a TECAN Infinite^{®} 200 PRO spectrophotometer (Tecan Italia srl).

The results presented in Figure 1 clearly show that mineralization had been activated in all groups, the values detected in the group treated with HYADD^{®}-4 however are much higher. HYADD^{®}-4 thus activates bone mineralization, stimulating and improving ossification.

The result is extraordinary and surprising as it was obtained with a concentration of HYADD^{®}-4 which is half of the concentration of linear HA used in Group 3.

As all the samples had been treated with osteoinductive medium, in order to be certain that the mineralization values depend on the effect of HYADD^{®}-4 and not on an increased number of cells, an MTT test was carried out (Mosmann T. J. Immunol. Methods; 1983, 65, 55-63) on samples identical to those prepared for the Alizarin Red S40 test. This test showed that the optical density, which is directly proportional to the metabolic activity of the cells and especially their number per sample, is absolutely comparable in all groups.

This therefore confirms that the treatment with HYADD^{®}-4 has no effect on the proliferation of the treated cells, but induces an important mineralization in mesenchymal cells differentiating into osteoblasts, significantly higher than that induced by linear HA and it is thus able to consistently stimulate and improve ossification. The result is even more relevant as it is obtained with a concentration of HYADD^{®}-4 approximately equal to half of the concentration of linear HA.

### EXAMPLE 2

### Evaluation of the influence of treatment on the gene expression during mineralization.

The expression of some genes relating to osteogenesis was assessed on cells prepared as for the experiment of Example 1 with respect to the isolation and culture phases, but subsequently treated as follows:
Group 1 (negative control): no treatment. The cells were simply kept in culture in the osteoinductive medium;
Group 2: the cells were treated with HYADD^{®}-4 1mg/mL;
Group 3: cells were treated with HA (PM 500-730kDa) 1 mg/mL.

The gene identified for the test is COL15A1: it encodes the synthesis of type XV collagen, which is found ubiquitously distributed in the body. Its function is typically to contribute to the grouping of the collagen fibril network, thus contributing to the extracellular bone matrix neoformation (Lisignoli et al., J. Cell. Mol. Med., 2017, 21, 2236-2244). Ultimately, an increase in the expression of COL15A1 is an index of the increase in the production of bone matrix in its entirety, fundamental for repair purposes.

### Materials and methods

Total RNA was extracted from the MSCs treated as described above, using TNA PURE Reagent (EuroClone SpA, Milan) at 24, 48, 72 hours. The samples were then treated with DNase I (DNA-Free Kit; Ambion, Life Technologies) and the quantification of RNA was effected with a Nanodrop^{®} spectrophotometer (EuroClone SpA, Milan). The reverse transcription was performed with Super Script^{®} Vilo^{™}cDNA Synthesis Kit (Invitrogen, Grand Island, NY, USA), following the manufacturer's instructions.

50µL of the cDNA obtained were mixed with "TaqMan^{®} Universal PCR Master Mix, No AmpErase UNG" (ThermoFisher Scientific), and then charged into the appropriate TaqMan cartridge suitably configured.

The RealTime PCR was carried out with the 7900HT Fast Real-Time PCR system (with TaqMan^{®} Array Block) (ThermoFisher Scientific), using universal cycle conditions (95°C/10 minutes, then [95°C/15 seconds, 60°C/60 seconds] for 40 cycles).

All the analyses of the results of the Real Time PCR were obtained with Thermofisher Cloud - Relative Quantification Software (Thermofisher Scientific).

For each target gene, the messenger RNA levels were calculated normalized to the ribosomal protein S9 (RPS9) according to formula 2-ΔCt, and expressed as a percentage of the reference gene.

The statistical analysis of the data was carried out using the General Linear Model (GLM).

The data obtained are indicated in Figure 2.

It is evident that the treatment with HYADD^{®}-4 induces the expression of the COL15A1 gene initially to a lesser extent with respect to the other treatments (from 7 to 14 days), but increases over time (from 14 to 28 days), far exceeding both the control values and, above all, the values of the treatment with linear HA which, after an initial peak, collapses to values even lower than those of the control.

HYADD^{®}-4 therefore induces the gene expression of COL15A1 lastingly over time, with a trend that continues to grow.

This result is extremely significant as such, and is even more significant considering that the regeneration process of the bone tissue as a whole is slow, particularly in the ossification and deposition phase of the extracellular matrix. The long-term increase in the expression of the COL15A1 gene means that the stimulus for the creation and organization of the extracellular bone matrix is maintained for a long time, thus ensuring a progressive and lasting consolidation process.

The treatment with HYADD^{®}-4 therefore represents a surprisingly effective therapeutic strategy in stimulating and maintaining the complex biological system necessary for bone mineralization, over time, thus bringing to an improvement and acceleration of ossification.

## Claims

1. Pharmaceutical composition in the form of hydrogels having a dynamic viscosity, measured at a temperature T of 20±0.5°C and at a shear rate of 1.0 sec⁻¹ , ranging from 55 to 75 Pa·s, said compositions consisting of one amide derivative of hyaluronic acid which is the hexadecyl amide prepared from a HA having a weight average MW ranging from 500 kDa to 730 kDa, and having an amidation degree ranging from 1% to 3% m/m, for use in the treatment of bone mineralization in bone traumas, preferably bone fractures, wherein the administration route is selected among local, locoregional, intra- or peri-osseous, with the exclusion of intraarticular administration and wherein the hexadecyl amide has a concentration ranging from 6 to 9 mg/ml, the pharmaceutical composition being formulated with the addition of pharmaceutically acceptable excipients.

2. Pharmaceutical composition for use according to claim 1, in the treatment of bone fractures, in patients suffering from pathologies **characterized by** a T-score index lower than -1.

3. Pharmaceutical composition for use according to claim 1, in the treatment of bone fractures, in patients suffering from pathologies **characterized by** a T-score index lower than -2.5.

4. Pharmaceutical composition for use according to claim 1, wherein the hexadecyl amide has a concentration equal to 8 mg/ml.

5. Pharmaceutical composition for use according to any of the previous claims, for the treatment of bone fractures in patients suffering from pathologies such as:
- diabetes, Gaucher disease, osteomalacia, rickets, postmenopausal primary or elderly osteoporosis, osteoporosis secondary to chronic drug intake, for example steroids, chemotherapy, immunosuppressants, GnRH analogues;
- hormonal imbalances and related diseases, for example, hypogonadism, hyperparatoroidism, Cushing's disease;
- avitaminosis and/or protein-calorie malnutrition and/or Malabsorption Syndrome.

## Patentansprüche

1. Pharmazeutische Zusammensetzung in Form von Hydrogelen mit einer dynamischen Viskosität, gemessen bei einer Temperatur T von 20±0,5 °C und einer Scherrate von 1,0 sec⁻¹, im Bereich von 55 bis 75 Pa·s, wobei die Zusammensetzungen aus einem Amidderivat der Hyaluronsäure bestehen, bei dem es sich um das Hexadecylamid handelt, das aus einem HA mit einem Gewichtsmittel MW im Bereich von 500 kDa bis 730 kDa und mit einem Amidierungsgrad im Bereich von 1 % bis 3 % m/m hergestellt wird, zur Verwendung bei der Behandlung der Knochenmineralisierung bei Knochentraumata, vorzugsweise Knochenbrüchen, wobei der Verabreichungsweg unter lokaler, lokoregionaler, intra- oder periossärer Verabreichung ausgewählt ist, mit Ausnahme der intraartikulären Verabreichung, und wobei das Hexadecylamid eine Konzentration im Bereich von 6 bis 9 mg/ml aufweist, wobei die pharmazeutische Zusammensetzung unter Zugabe von pharmazeutisch verträglichen Hilfsstoffen formuliert ist.

2. Pharmazeutische Zusammensetzung zur Verwendung nach Anspruch 1 bei der Behandlung von Knochenbrüchen bei Patienten, die an Pathologien leiden, die durch einen T-Score-Index von weniger als -1 gekennzeichnet sind.

3. Pharmazeutische Zusammensetzung zur Verwendung nach Anspruch 1 bei der Behandlung von Knochenbrüchen bei Patienten, die an Pathologien leiden, die durch einen T-Score-Index von weniger als -2,5 gekennzeichnet sind.

4. Pharmazeutische Zusammensetzung zur Verwendung nach Anspruch 1, wobei das Hexadecylamid eine Konzentration von 8 mg/ml aufweist.

5. Pharmazeutische Zusammensetzung zur Verwendung nach einem der vorhergehenden Ansprüche zur Behandlung von Knochenbrüchen bei Patienten mit Pathologien wie:
- Diabetes, Morbus Gaucher, Osteomalazie, Rachitis, primäre Osteoporose nach der Menopause oder bei älteren Menschen, Osteoporose infolge chronischer Medikamenteneinnahme, z. B. Steroide, Chemotherapie, Immunsuppressiva, GnRH-Analoga;
- hormonelle Ungleichgewichte und verwandte Krankheiten, z. B. Hypogonadismus, Hyperparatoroidismus, Morbus Cushing;
- Avitaminose und/oder Protein-Kalorien-Mangelernährung und/oder Malabsorptionssyndrom.

## Revendications

1. Composition pharmaceutique sous forme d'hydrogels ayant une viscosité dynamique, mesurée à une température T de 20±0,5°C et à un taux de cisaillement de 1,0 sec-1, allant de 55 à 75 Pa-s, lesdites compositions consistant en un dérivé amide de l'acide hyaluronique qui est l'hexadécylamide préparé à partir d'un AH ayant un MW moyen en poids allant de 500 kDa à 730 kDa, et ayant un degré d'amidation allant de 1 % à 3 % m/m, pour une utilisation dans le traitement de la minéralisation osseuse dans les traumatismes osseux, de préférence les fractures osseuses, dans laquelle la voie d'administration est choisie parmi les voies locales, locorégionales, intra- ou péri-osseuses, à l'exclusion de l'administration intra-articulaire, et la concentration de l'hexadécylamide est comprise entre 6 et 9 mg/ml, la composition pharmaceutique étant formulée avec l'ajout d'excipients pharmaceutiquement acceptables.

2. Composition pharmaceutique pour une utilisation selon la revendication 1, dans le traitement des fractures osseuses, chez les patients souffrant de pathologies **caractérisées par** un indice T-score inférieur à -1.

3. Composition pharmaceutique pour une utilisation selon la revendication 1, dans le traitement des fractures osseuses, chez les patients souffrant de pathologies **caractérisées par** un indice T-score inférieur à -2,5.

4. Composition pharmaceutique selon la revendication 1, dans laquelle l'hexadécylamide a une concentration égale à 8 mg/ml.

5. Composition pharmaceutique pour une utilisation selon l'une quelconque des revendications précédentes, pour le traitement des fractures osseuses chez les patients souffrant de pathologies telles que :
- le diabète, la maladie de Gaucher, l'ostéomalacie, le rachitisme, l'ostéoporose primaire post-ménopausique ou chez les personnes âgées, l'ostéoporose secondaire à la prise chronique de médicaments, par exemple stéroïdes, chimiothérapie, immunosuppresseurs, analogues de la GnRH ;
- les déséquilibres hormonaux et les maladies associées, par exemple l'hypogonadisme, l'hyperparatoroïdisme, la maladie de Cushing ;
- l'avitaminose et/ou la malnutrition protéino-calorique et/ou le syndrome de malabsorption.
